# FASCICULE DE BREVET EUROPEEN

(11) **EP 1 196 185 B1**
(45) Date de publication et mention de la délivrance du brevet: **09.03.2005**
(21) Numéro de dépôt: 00958610.8
(22) Date de dépôt: 25.07.2000
(51) Int. Cl.: A61K 38/05, A61K 33/14, A61P 17/00

(54) **NOUVELLE UTILISATION DE SOLUTIONS SALINES HYPOOSMOTIQUES ET MEDICAMENT A BASE DE CES SOLUTIONS**
NEUE VERWENDUNG VON HYPOOSMOTISCHEN SALINELÖSUNGEN UND DARAUF BASIERENDEM MEDIKAMENT
NOVEL USE OF HYPO-OSMOTIC SALINE SOLUTIONS AND MEDICINE BASED ON SAID SOLUTIONS

(30) Priorité: 27.07.1999 FR 9909743
(43) Date de publication de la demande: 17.04.2002
(73) Titulaire: LABORATOIRES GOEMAR S.A., 35400 Saint-Malo (FR)
(72) Inventeur: YVIN, Jean-Claude, F-35400 Saint Malo (FR); HALLEY, Bénédicte, F-14000 Caen (FR)
(74) Mandataire: Koch, Gustave
(86) Numéro de dépôt international: PCT/FR2000/002135
(87) Numéro de publication internationale: WO 2001/007073

(56) Documents cités:
- WO-A-00/01395
- CN-A- 1 129 099
- DE-A- 4 339 750
- FR-A- 2 778 562
- DATABASE WPI Section Ch, Week 199324 Derwent Publications Ltd., London, GB; Class D21, AN 1993-191439 XP002138626 & JP 05 117158 A (SASAKI KAGAKU YAKUHIN KK), 14 mai 1993 (1993-05-14)

## Description

L'invention a pour objet une nouvelle utilisation de solutions salines hypoosmotiques préparées à partir d'eau de mer, pour l'obtention d'un médicament.

Elle vise également un médicament à base de ces solutions salines hypoosmotiques, destiné à un traitement pour la prévention et la limitation de la libération des médiateurs chimiques responsables du déclenchement des phénomènes inflammatoires de la peau.

Les médiateurs chimiques en question sont libérés dans le cas de certaines maladies de la peau de même que sous l'action des composants de certains produits cosmétiques, notamment de certains produits destinés aux soins de la peau.

Ainsi, dans le cas d'affections du groupe comprenant les réactions allergiques, à savoir l'eczéma (hypersensibilité retardée) et l'urticaire (hypersensibilité immédiate), le psoriasis, les dermites irritatives en général, la dermatite atopique, les brûlures et le prurigo, il se produit, au niveau de l'épiderme notamment, une libération plus ou moins importante de médiateurs chimiques, dont l'interleukine IL-8, qui sont à l'origine du déclenchement de phénomènes inflammatoires dont la peau est le siège et qui peuvent devenir très gênants pour le patient.

Ces phénomènes peuvent également se produire en cas d'érythème solaire ou coup de soleil et dans le cas de piqûres d'insectes ou de végétaux.

Il en est de même chez certains sujets en bonne santé mais dont la peau est sensible, irritée ou réactive lorsque cette peau est mise au contact de certains produits cosmétiques dont des constituants, pas toujours identifiés comme tels, jouent le rôle d'agents pro-inflammatoires au niveau de la peau et, jouant ce rôle, provoquent la libération des médiateurs chimiques qui, à leur tour, déclenchent des phénomènes inflammatoires se traduisant le plus souvent par des rougeurs, notamment au visage.

Il est déjà connu de lutter contre toutes ces pathologies en ayant recours à des médicaments à base de corticostéroïdes tels que la dexaméthasone ou de substances anti-inflammatoires non stéroïdiénnes telles que le bufexamac.

Les résultats obtenus avec les corticostéroïdes et les substances anti-inflammatoires non stéroïdiennes sont satisfaisants mais il est bien connu que ces produits ont des effets secondaires quelquefois gênants.

L'invention a pour but, surtout, de remédier aux inconvénients de l'art antérieur et de fournir pour le traitement des phénomènes inflammatoires en question, un médicament exempt d'effets secondaires.

Et la Société Demanderesse a le mérite d'avoir trouvé, à l'issue de travaux de recherche approfondis, que ce but était atteint en ayant recours à un médicament obtenu par l'utilisation de solutions salines hypoosmotiques préparées à partir d'eau de mer, et destiné à un traitement propre à prévenir et à limiter la libération --tant dans le cas des maladies du groupe défini plus haut que sous l'action des constituants de certains produits cosmétiques, ces constituants jouant le rôle d'agents pro-inflammatoires-- des médiateurs chimiques responsables du déclenchement des phénomènes inflammatoires cutanés.

Les solutions salines hypoosmotiques préparées à partir d'eau de mer, en question présentent :
- un pH de 5,0 à 8,30,
- une résistivité de 52 à 370 Ω et de préférence de 57 à 85 Ω,
- une densité de 1,002 à 1,008,
- une teneur en matière sèche de 0,2% à 0,9%,
- une osmolarité comprise entre 100 mOs et 305 mOs/kg (milliosmoles/kg), de préférence entre 140 et 240 mOs/kg, et
- une constitution chimique dont les principaux éléments résultent du tableau I ci-après:

**TABLEAU I**

| | |
|---|---|
| Sodium (Na) | de 600 à 2000 mg/l |
| Potassium (K) | de 6 à 40 mg/l |
| Chlorures (Cl) | de 2000 à 5800 mg/l |
| Calcium (Ca) | de 200 à 300 mg/l |
| Magnésium (Mg) | de 1000 à 1200 mg/l |
| Sulfates (SO₄) | de 2000 à 3000 mg/l |

En conséquence, l'invention réside dans l'utilisation de solutions salines hypoosmotiques, préparées à partir d'eau de mer, pour l'obtention d'un médicament destiné à un traitement des phénomènes inflammatoires cutanés se traduisant le plus souvent par des rougeurs notamment au visage en prévenant et limitant la libération des médiateurs chimiques responsables du déclenchement desdits phénomènes inflammatoires cutanés.

Plus particulièrement, l'invention vise l'utilisation, pour l'obtention d'un médicament destiné à un traitement des phénomènes inflammatoires cutanés se traduisant le plus souvent par des rougeurs notamment au visage en prévenant et limitant la libération des médiateurs chimiques responsables du déclenchement desdits phénomènes inflammatoires cutanés, d'une solution saline hypoosmotique présentant,
- un pH de 5,0 à 8,30,
- une résistivité de 52 à 370 Ω et de préférence de 57 à 85 Ω,
- une densité de 1,002 à 1,008,
- une teneur en matière sèche de 0,2% à 0,9%,
- une osmolarité comprise entre 100 mOs et 305 mOs/kg (milliosmoles/kg), de préférence entre 140 et 240 mOs/kg, et
- une constitution chimique dont les principaux éléments résultent du tableau I ci-après:

**TABLEAU I**

| | |
|---|---|
| Sodium (Na) | de 600 à 2000 mg/l |
| Potassium (K) | de 6 à 40 mg/l |
| Chlorures (Cl) | de 2000 à 5800 mg/l |
| Calcium (Ca) | de 200 à 300 mg/l |
| Magnésium (Mg) | de 1000 à 1200 mg/l |
| Sulfates (SO₄) | de 2000 à 3000 mg/l |

Pour préparer les solutions hypoosmotiques, utilisées conformément à l'invention, on peut procéder comme indiqué ci-après.

On a recours, en tant que matière première, à une eau de mer prélevée de préférence par une profondeur de 5 à 10 mètres dans une zone à forts mouvements de courant, et caractérisée par une teneur en sels supérieure à 32 g/l.

Cette eau est analysée, décantée puis rapidement :
- désodée par la technique d'électrodialyse jusqu'à ce que l'osmolarité soit amenée à une valeur comprise entre 100 et 305 mOs/kg,
- filtrée,
- stockée dans des conditions stériles, notamment en cuve en acier inoxydable.

Elle est de nouveau analysée à ce stade pour vérifier :
- sa stérilité,
- son osmolarité.

Enfin, elle peut être conditionnée de façon stérile dans un local spécialement traité en atmosphère contrôlée.

Les travaux qui sont à la base de l'invention comportent des expériences montrant que les solutions salines hypoosmotiques utilisées conformément à l'invention, tout comme les solutions salines isoosmotiques bien connues et comme certains corticostéroïdes tels que le dexaméthasone, ne sont pas cytotoxiques à l'égard des kératinocytes alors que leur capacité à prévenir et à limiter la libération sous l'influence de certaines affections ou de certains agents pro-inflammatoires, des médiateurs chimiques responsables du déclenchement des phénomènes inflammatoires cutanés est supérieure à celle des deux produits de référence qui viennent d'être cités.

Pour l'étude de la toxicité, on a utilisé des kératinocytes épidermiques humains normaux issus de prélèvements épidermiques ; il s'agit plus particulièrement de kératinocytes isolés à partir du résidu opératoire d'une plastie abdominale réalisée chez une femme âgée de 27 ans (sujet I0020).

On a cultivé ces kératinocytes à 37°C dans un milieu de culture pour kératinocytes ou MCK constitué par celui commercialisé sous la désignation « SFM defined-keratinocyte médium » par la Société GIBCO sous une atmosphère humide contenant 5% de CO₂, jusqu'à confluence des monocouches.

Les produits testés étaient constitués par
■ trois solutions salines hypoosmotiques désignées par J, I et H dont les résistivités sont respectivement de 57,30 Ω, 64, 93 Ω et de 74,34 Ω èt dont les principaux éléments constitutifs apparaissent dans le tableau II ci-après:

**TABLEAU II**

| Constituants | Unités | H | I | J |
|---|---|---|---|---|
| Sodium (Na) | mg/l | 765 | 1130 | . 1370 |
| Potassium (K) | mg/l | 11,2 | 20,8 | 27,4 |
| Chlorures (Cl) | mg/l | 2930 | 3797 | 4248 |
| Calcium (Ca) | mg/l | 290 | 310 | 330 |
| Magnésium (Mg) | mg/l | 1130 | 1200 | 1200 |
| Sulfates (SO₄) | mg/l | 2570 | 2570 | 2600 |

■ la solution isoosmotique commercialisée par la Société Demanderesse sous la marque de fabrique « PHYSIOMER », désignée par K,
■ la dexaméthasone et
■ le sérum physiologique commercialisé sous la marque BABISOL.

On a incubé ces six produits pendant 4 heures dans les cultures de kératinocytes.

Avant l'incubation, les cellules, c'est-à-dire les kératinocytes, ont été rincées avec les produits essayés afin d'éliminer toute trace de milieu de culture.

Les solutions salines J, I, H et K et le sérum physiologique ont été utilisées non diluées.

La dexaméthasone a été testée à 10 µM dans le milieu MCK.

Les cultures de kératinocytes ont été incubées en présence des six produits pendant 4 heures à 37°C, dans une atmosphère humide contenant 5% de CO₂.

Des cultures témoins, incubées dans le milieu MCK en absence des six produits, ont été réalisées en parallèle.

Les essais ont été réalisés en triple.

L'effet cytotoxique des six produits essayés a été apprécié par la détermination du pourcentage de protéines subsistant dans les kératinocytes après 4 heures d'incubation en présence des différents produits, étant entendu que, lorsque les kératinocytes sont incubées dans le milieu de culture MCK, la perte en protéines est nulle, les kératinocytes conservant donc 100% de leurs protéines.

Les pourcentages de protéines subsistant en présence des six produits testés sont réunis dans le tableau III ci-après.

**TABLEAU III**

| Témoin MCK | Dexaméthasone 10 µM dans MCK | Sérum physiologique | J | I | H | K |
|---|---|---|---|---|---|---|
| 100 | 89 | 32 | 86 | 73 | 86 | 86 |
| Les résultats sont exprimés en mg de protéines/ml. | | | | | | |

De l'examen des valeurs réunies dans le tableau III, il résulte :
■ que le sérum physiologique est fortement cytotoxique,
■ que la dexaméthasone et les produits J, H et K sont équivalents et très peu cytotoxiques, la solution saline hypoosmotique I étant un peu moins satisfaisante de ce point de vue.

On a ensuite étudié la capacité des solutions salines hypoosmotiques, utilisées conformément à l'invention, à prévenir et à limiter la libération, sous l'influence de certaines affections ou certains agents pro-inflammatoires, des médiateurs chimiques responsables du déclenchement des phénomènes inflammatoires cutanés.

Le médiateur chimique étudié était l'interleukine IL-8.

L'agent pro-inflammatoire était le milieu de culture MCK qui s'est révélé être à l'origine d'une forte libération d'interleukine IL-8 dans les cultures de kératinocytes.

On a procédé d'une manière semblable à celle retenue pour les essais décrits plus haut et relatifs à l'appréciation de la cytotoxicité des différents produits testés.

Ces produits étaient les mêmes.

La durée de l'incubation était également de 4 heures.

Sous l'influence du milieu MCK, la libération d'interleukine IL-8 est de 100 pg/mg de protéines ; on a dosé dans ce même milieu les quantités d'interleukine IL-8, libérée en présence des six produits testés, à savoir, d'une part, les produits J, I, H, K, ainsi que le sérum physiologique qui ont été utilisés non dilués et, d'autre part, la dexaméthasone qui a été utilisée à raison de 10 µM dans le milieu MCK.

Les valeurs ainsi trouvées sont réunies dans le tableau IV ci-après.

**TABLEAU IV**

| Témoin MCK | Dexaméthasone 10 µM dans MCK | Sérum physiologique | J | I | H | K |
|---|---|---|---|---|---|---|
| 100 | 74 | 165 | 40 | 42 | 50 | 89 |
| Les résultats sont exprimés en pg d'IL-8/mg de protéines. | | | | | | |

De l'examen des valeurs réunies dans le tableau IV, il apparaît :
■ que le sérum physiologique a pour effet d'augmenter la libération d'IL-8,
■ que la solution saline isoosmotique K limite légèrement la libération de l'IL-8 et que la dexaméthasone la limite un peu plus mais, surtout,
■ que les solutions salines hypoosmotiques J, I et H limitent cette libération très fortement.

L'invention vise l'utilisation, pour l'obtention d'un médicament destiné à un traitement des phénomènes inflammatoires cutanés se traduisant le plus souvent par des rougeurs notamment au visage en prévenant et limitant la libération des médiateurs chimiques responsables du déclenchement desdits phénomènes inflammatoires cutanés, de solutions hypoosmotiques désignées par J, I et H dont les résistivités sont respectivement de 57,30 Ω, 64,93 Ω et de 74,34 Ω et dont les principaux éléments constitutifs apparaissent dans le tableau II ci-après:

**TABLEAU II**

| Constituants | Unités | H | I | J |
|---|---|---|---|---|
| Sodium (Na) | mg/l | 765 | 1130 | 1370 |
| Potassium (K) | mg/l | 11,2 | 20,8 | 27,4 |
| Chlorures (Cl) | mg/l | 2930 | 3797 | 4248 |
| Calcium (Ca) | mg/l | 290 | 310 | 330 |
| Magnésium (Mg) | mg/l | 1130 | 1200 | 1200 |
| Sulfates (SO₄) | mg/l | 2570 | 2570 | 2600 |

Du point de vue pratique, les médicaments obtenus par l'utilisation des solutions salines hypoosmotiques en vue du traitement de prévention et de limitation de la libération des médiateurs chimiques responsables du déclenchement des phénomènes inflammatoires cutanés peuvent se présenter sous la forme
- d'émulsions (crèmes, laits, émulsions multiples, micro-émulsions, nano-émulsions et autres),
- de gels (aqueux ou huileux),
- de pâtes (dentifrices, masques et autres),
- de solutions (aqueuses ou huileuses), c'est-à-dire de lotions et autres,
- de suspensions et
- de dispositifs transdermiques.

A titre illustratif, on indique ci-après deux exemples de tels médicaments, à savoir une crème anti-prurit et une pâte dentifrice efficace contre l'inflammation gingivale.

| **1. Crème anti-prurit** | |
|---|---|
| Eau déminéralisée | 44,8% |
| Glycérine | 5 % |
| Solution (I) d'eau de mer à 7 g/l | 20 % |
| Laureth-9 | 1,5% |
| PEG-100 Stéarate glycéryl stéarate | 6 % |
| Alcool cétylique | 2 % |
| Isononyl isonanoate | 2 % |
| Cétéaryl octanoate | 8 % |
| Hexyldécyl stéarate | 6,5% |
| Cyclométhicone | 2 % |
| Conservateur | 1 % |
| Polyacrylamide isoparaffine laureth-7 | 1,2%. |

| **2. Pâte dentifrice efficace contre l'inflammation gingivale** | |
|---|---|
| Solution (I) d'eau de mer à 7 g/l | 45,9% |
| Carbonate de calcium | 30,9% |
| Sorbitol | 8 % |
| Monofluorophosphate de sodium | 0,8% |
| Carraghénane | 2 % |
| Silice hydratée | 1 % |
| Lithothamne | 11 % |
| Arôme | 0,3% |
| Conservateur | 0,1%. |

Pour lutter contre les effets secondaires quelquefois gênants qui surviennent chez des personnes particulièrement sensibles lors de l'utilisation de certains produits cosmétiques, on peut incorporer dans ces produits cosmétiques le médicament obtenu par l'utilisation des solutions salines hypoosmotiques préparées à partir d'eau de mer, en vue du traitement de prévention et de limitation de la libération des médiateurs chimiques responsables du déclenchement des phénomènes inflammatoires cutanés.

L'invention vise donc également l' utilisation autre qu'une méthode de traitement thérapeutique des solutions salines hypoosmotiques selon l'une des revendications 1 à 3 pour le traitement cosmétique des rougeurs cutanées bénignes.

Il est vrai que, dans le cas des produits cosmétiques, les phénomènes inflammatoires dont il vient d'être question se limitent la plupart du temps à quelques rougeurs cutanées.

Mais même ces manifestations la plupart du temps bénignes sont mal ressenties par les personnes concernées.

Pour y remédier, on incorpore donc les solutions salines hypoosmotiques, préparées à partir d'eau de mer en question dans les produits cosmétologiques en rapport avec lesquels les phénomènes en question ont été constatés.

L'invention vise donc aussi une utilisation autre qu'une méthode de traitement thérapeutique des solutions salines hypoosmotiques préparées à partir d'eau de mer définies plus haut pour le traitement cosmétique des rougeurs cutanées bénignes.

A titre illustratif, on donne ici deux exemples de produits cosmétiques comportant lesdits médicaments, à savoir une lotion calmante et un lait démaquillant, tous deux pour peaux sensibles.

| **3. Lotion calmante pour peaux sensibles** | |
|---|---|
| Solution hypotonique(J) d'eau de mer à 8 g/l | 91,3% |
| Pyrrolidone carboxylate de sodium | 3,0% |
| Extrait d'algues | 2,0% |
| Polysorbate 20 | 2,0% |
| Glycérine | 1,0% |
| Conservateur | 0,7% |

| **4. Lait démaquillant pour peaux sensibles** | |
|---|---|
| Eau purifiée | 60,9% |
| Solution (H) d'eau de mer hypotonique à 6 g/l | 18,6% |
| Extrait d'algues | 6 % |
| Propylène glycol | 3 % |
| Alcool cétylique | 2,5% |
| Sorbitol | 2 % |
| Glycéryl stéarate | 1,5% |
| Ethers d'acides gras | 3,45% |
| Alcool stéarylique | 1,05% |
| Conservateur | 1 % |
| Hydroxyde de sodium | qsp pH = 6 |
| Sorbitol | 2 % |
| Glycéryl stéarate | 1,5% |
| Ethers d'acides gras | 3,45% |
| Alcool stéarylique | 1,05% |
| Conservateur | 1 % |
| Hydroxyde de sodium | qsp pH = 6. |

## Revendications

1. Utilisation de solutions salines hypoosmotiques, préparées à partir d'eau de mer, pour l'obtention d'un médicament destiné à un traitement des phénomènes inflammatoires cutanés se traduisant le plus souvent par des rougeurs notamment au visage en prévenant et limitant la libération des médiateurs chimiques responsables du déclenchement desdits phénomènes inflammatoires cutanés.

2. Utilisation selon la revendication 1, d'une solution saline hypoosmotique présentant :
- un pH de 5,0 à 8,30,
- une résistivité de 52 à 370 Ω et de préférence de 57 à 85 Ω,
- une densité de 1,002 à 1,008,
- une teneur en matière sèche de 0,2% à 0,9%,
- une osmolarité comprise entre 100 mOs et 305 mOs/kg (milliosmoles/kg), de préférence entre 140 et 240 mOs/kg, et
- une constitution chimique dont les principaux éléments résultent du tableau I ci-après:
**TABLEAU I**
| | |
|---|---|
| Sodium (Na) | de 600 à 2000 mg/l |
| Potassium (K) | de 6 à 40 mg/l |
| Chlorures (Cl) | de 2000 à 5800 mg/l |
| Calcium (Ca) | de 200 à 300 mg/l |
| Magnésium (Mg) | de 1000 à 1200 mg/l |
| Sulfates (SO₄) | de 2000 à 3000 mg/l |

3. Utilisation selon l'une des revendications 1 et 2, de solutions hypoosmotiques désignées par J, I et H dont les résistivités sont respectivement de 57,30 Ω, 64,93 Ω et de 74,34 Ω et dont les principaux éléments constitutifs apparaissent dans le tableau II ci-après:
**TABLEAU II**
| Constituants | Unités | H | I | J |
|---|---|---|---|---|
| Sodium (Na) | mg/l | 765 | 1130 | 1370 |
| Potassium (K) | mg/l | 11,2 | 20,8 | 27,4 |
| Chlorures (Cl) | mg/l | 2930 | 3797 | 4248 |
| Calcium (Ca) | mg/l | 290 | 310 | 330 |
| Magnésium (Mg) | mg/l | 1130 | 1200 | 1200 |
| Sulfates (SO₄) | mg/l | 2570 | 2570 | 2600 |

4. Utilisation autre qu'une méthode de traitement thérapeutique des solutions salines hypoosmotiques selon l'une des revendications 1 à 3 pour le traitement cosmétique des rougeurs cutanées bénignes.

## Patentansprüche

1. Verwendung von aus Meerwasser hergestellten hypoosmotischen Salzlösungen zur Gewinnung eines Medikaments, das zu einer Behandlung von entzündlichen Erscheinungen der Haut bestimmt ist, die sich meist durch Rötungen, insbesondere im Gesicht, äußern, durch Verhindern und Begrenzen der Freisetzung von für die Auslösung dieser entzündlichen Erscheinungen der Haut verantwortlichen chemischen Mediatoren.

2. Verwendung nach Anspruch 1 einer hypoosmotischen Salzlösung mit:
- einem pH von 5,0 bis 8,30,
- einem spezifischen Widerstand von 25 - 37 Ω und vorzugsweise von 57 bis 85 Ω,
- einer Dichte von 1,002 bis 1,008,
- einem Trockenmassegehalt von 0,2 bis 0,9 %
- einer Osmolarität zwischen 100 mOs und 305 mOs/kg (Milliosmol/kg), vorzugsweise zwischen 140 und 240 mOs/kg und
- einer chemischen Zusammensetzung, deren wesentliche Elemente sich aus nachfolgender Tabelle I ergeben:
**Tabelle I**
| | |
|---|---|
| Natrium (Na) | von 600 bis 2000 mg/l |
| Kalium (K) | von 6 bis 40 mg/l |
| Chloride (Cl) | von 2000 bis 5800 mg/l |
| Calcium (Ca) | von 200 bis 300 mg/l |
| Magnesium (Ma) | von 1000 bis 1200 mg/l |
| Sulfate (SO₄) | von 2000 bis 3000 mg/l |

3. Verwendung nach einem der Anspüche 1 und 2 von mit J, I und H bezeichneten hypoosmotischen Lösungen, deren spezifische Wiederstände 57,30 Ω, 64,93 Ω, und 74,34 Ω betragen und deren wesentliche Bestandteile sich aus nachfolgender Tabelle II ergeben:
**Tabelle II**
| Bestandteile | Einheiten | H | I | J |
|---|---|---|---|---|
| Natrium (Na) | Mg/l | 765 | 1130 | 1370 |
| Kalium (K) | Mg/l | 11,2 | 20,8 | 27,4 |
| Chloride (Cl) | Mg/l | 2930 | 3797 | 4248 |
| Calcium (Ca) | Mg/l | 290 | 310 | 330 |
| Magnesium (Mg) | Mg/l | 1130 | 1200 | 1200 |
| Sulfate (SO₄) | Mg/l | 2570 | 2570 | 2600 |

4. Nichttherapeutische Verwendung von hypoosmotischen Salzlösungen nach einem der Ansprüche 1 bis 3 zur kosmetischen Behandlung von gutartigen Hautrötungen.

## Claims

1. Use of hypoosmotic saline solutions prepared starting from sea-water for the obtention of a medicine intended for a treatment of the cutaneous inflammatory phenomena, which express themselves most frequently by redness or blush especially on the face, by preventing and limitating the release of the chemical mediators which are responsible for the starting of the said cutaneous inflammatory phenomena.

2. Treatment according to claim 1, of a hypoosmotic saline solution having:
- a pH of from 5,0 to 8,30,
- a resistivity of from 52 to 370 Ω and preferably from 57 to 85 Ω,
- a density of from 1,002 to 1,008,
- a dry matter content of from 0,2 to 0,9%,
- an osmolarity comprised between 100 mOs and 305 mOs/kg (milliosmols/kg), preferably between 140 and 240 mOs/kg, and
- a chemical constitution whose principal constituents appear from Table 1 hereafter:
**TABLE I**
| | |
|---|---|
| Sodium (Na) | from 600 to 2000 mg/l |
| Potassium (K) | from 6 to 40 mg/l |
| Chlorures (Cl) | from 2000 to 5800 mg/l |
| Calcium (Ca) | from 200 to 300 mg/l |
| Magnesium (Mg) | from 1000 to 1200 mg/l |
| Sulfate (SO₄) | from 2000 to 3000 mg/l |

3. Use according to one of claims 1 and 2, of hypoosmotic solutions denoted J, I and H, whose resistivities are respectively equal to 57,30 Ω, 64,93 Ω and 74,34 Ω and whose principal constituting elements appear from the following Table II:
**TABLE II**
| **Constituents** | **Units** | **H** | **I** | **J** |
|---|---|---|---|---|
| **Sodium (Na)** | mg/l | 765 | 1130 | 1370 |
| **Potassium (K)** | mg/l | 11,2 | 20,8 | 27,4 |
| **Chlorides (Cl)** | mg/l | 2930 | 3797 | 4248 |
| **Calcium (Ca)** | mg/l | 290 | 310 | 330 |
| **Magnesium(Mg)** | mg/l | 1130 | 1200 | 1200 |
| **Sulfates (SO**_{**4**}**)** | mg/l | 2570 | 2570 | 2600 |

4. Use other than a therapeutical treatment of the saline hypoosmotic solutions according to one of claims 1 to 3 for the cosmetic treatment of benign cutaneous redness or blush.
